## Europäisches Patentamt
## European Patent Office
## Office européen des brevets

(11) Veröffentlichungsnummer: **0 189 910**
**B1**

(12) # EUROPÄISCHE PATENTSCHRIFT

(45) Veröffentlichungstag der Patentschrift:
23.06.89

(21) Anmeldenummer: 86101150.0

(22) Anmeldetag: 29.01.86

(51) Int. Cl.⁴: **C 12 Q 1/56 // C12Q1/48, C12Q1/38**

(54) Verfahren zur Bestimmung des fibrinolytischen Zustands von Plasma.

(30) Priorität: 29.01.85 DE 3502878

(43) Veröffentlichungstag der Anmeldung:
06.08.86 Patentblatt 86/32

(45) Bekanntmachung des Hinweises auf die Patenterteilung:
23.08.89 Patentblatt 89/34

(84) Benannte Vertragsstaaten:
AT BE CH DE FR GB IT LI LU NL SE

(56) Entgegenhaltungen:
EP-A-0 137 269
FR-A-2 238 933
GB-A-1 551 064

PATENT ABSTRACTS OF JAPAN, Band 9, Nr. 78 (C-274) 1801 , 6. April 1985; & JP - A - 59 210 900 (HISAHARU MIHAMA) 29.11.1984
CLINICAL CHEMISTRY, Band 28, Nr. 5, Mai 1982, Seiten 1125-1128, Washington, US; E.E. CAMPBELL et al.: "A colorimetric assay for releasable plasminogen activator"
CLINICAL CHEMISTRY, Band 26, Nr. 10, September 1980, Seiten 1380-1391, Easton, Pennsylvania, US; J. FAREED et al.: "New perspectives in coagulation testing"
CLINICAL CHEMISTRY, Band 31, Nr. 9, September 1985, Seiten 1468-1473, Winston-Salem, NC, US; R.S. SCHIFREEN et al.: "A quantitative automated

(73) Patentinhaber: BOEHRINGER MANNHEIM GMBH, Patentabteilung, Abt. E Sandhofer Strasse 112-132 Postfach 31 01 20, D-6800 Mannheim 31 Waldhof (DE)

(72) Erfinder: Bartl, Knut, Dr.rer.nat., Am Westend 6, D-8121 Wilzhofen (DE)
Erfinder: Lill, Helmut, Dr.rer.nat., Zugspitzstrasse 24, D-8121 Wielenbach (DE)

(74) Vertreter: Weickmann, Heinrich, Dipl.- Ing., Patentanwälte Dipl.- Ing. H.Weickmann Dipl.- Phys.Dr. K.Fincke Dipl.- Ing. F.A.Weickmann Dipl.- Chem. B. Huber Dr.- Ing. H. Liska Dipl.- Phys.Dr. J. Prechtel Postfach 860820, D-8000 München 86 (DE)

(56) Entgegenhaltungen: (Fortsetzung)
immunoassay for fibrinogen/fibrin degradation products"

**Beschreibung**

Fibrinolyse ist die gegenläufige Reaktion der Blutgerinnung. Bei der Blutgerinnung entsteht aus Fibrinogen unter dem Einfluß des Enzyms Thrombin Fibrinmonomer, welches unter dem Einfluß von Kalziumionen und anderen Faktoren zu einem unlöslichen Gerüst von Fibrin polymerisiert. Die entstehenden Fibrinmoleküle bewirken eine Verfestigung von Blutgerinnseln. Da dieser Vorgang der Fibrinbildung im Gefäßsystem ständig abläuft, ist ein gegenläufiges System, das fibrinolytische System, ebenfalls ständig aktiv um zu verhindern, daß es ohne besonderen Anlaß zur Bildung von Thromben kommt. Dieses fibrinolytische System führt unter dem Einfluß von Plasminogenaktivator zur Bildung von Plasmin aus Plasminogen und Plasmin bewirkt die Auflösung von Fibrin unter Bildung von Fibrinspaltprodukten. Die Bestimmung des fibrinolytischen Zustands eines Plasmas ist daher von großer Bedeutung für die Beurteilung von Thrombosegefahren.

Es sind bereits Testmethoden bekannt, welche die spontane fibrinolytische Aktivität des Blutes (des Plasmas) bzw. die fibrinolytische Reaktion auf Stimulantien, welche Plasminogenaktivator aus der Gefäßwand freisetzen, messen. Hierbei ist es erforderlich, die Einwirkung von Plasmainhibitoren auszuschalten, was in der Praxis jedoch meist nur unzulänglich gelingt und gleichzeitig auch eine artifizielle Veränderung der Probe bedeutet.

Bei der Bestimmung der Euglobulin-Lysezeit geht man von Citratplasma aus. Dieses wird mit Essigsäure versetzt und abzentrifugiert. Der Niederschlag wird in Puffer gelöst und mit Thrombin versetzt. Es wird bei 37°C inkubiert und die Zeit bis zur Auflösung des Gerinnsels gemessen. Die Meßzeit wird hierdurch zwar wesentlich verkürzt, man bestimmt hierbei jedoch eine in vitro Fibrinolyseaktivität, die nicht notwendigerweise mit der in vivo Aktivität korreliert.

Man hat daher auch schon Verfahren entwickelt, die ohne Eliminierung der Plasmainhibitoren durchgeführt werden. Eine solche Methode ist z. B. die Bestimmung der Vollblut-Lysezeit.

So wird bei Bestimmung der Vollblutlysezeit Vollblut mit Thrombin versetzt und das gebildete Gerinnsel bei 37°C inkubiert und die Zeit bis zu seiner Auflösung gemessen. Diese Zeit beträgt bei Normalwerten nicht unter 24 Stunden. Die Methode ist daher zeitaufwendig, nicht automatisierbar und nur zur groben Abschätzung des fibrinolytischen Zustands geeignet. Eine abnormal niedrige fibrinolytische Aktivität kann gar nicht bestimmt werden.

Etwas kürzere Zeiten ergeben sich, wenn mit verdünntem Blut gearbeitet wird, die erwähnten Nachteile bleiben jedoch im übrigen bestehen.

Genaue Ergebnisse sollte der sogenannte Fibrinplattentest liefern, der mit Citratplasma oder resuspendierten Euglobulinniederschlägen arbeitet. Hauptnachteil liegt hier in der langen Inkubationszeit von 18 Stunden bei 37°C.

Aus Clin. Chem. Vol. 26, Nr. 10, 1980, Seiten 1380 bis 1390 war es bekannt, einzelne Faktoren der Gerinnungskaskade über jeweils spezifische Immunoassays zu bestimmen.

Der Erfindung liegt daher die Aufgabe zugrunde, einen Fibrinolyseglobaltest zu schaffen, welcher die Nachteile der oben erwähnten bekannten Methoden vermeidet und insbesondere in relativ kurzer Zeit Ergebnisse liefert, welche den in vivo Zustand zuverlässig wiederspiegeln, der sich automatisieren läßt und photometrisch ausgewertet werden kann.

Gelöst wird diese Aufgabe erfindungsgemäß durch ein Verfahren zur Bestimmung des fibrinolytischen von Zustands Plasma, welches dadurch gekennzeichnet ist, daß man einer nativen Plasmaprobe

a) eine zur Trübungsbildung ausreichende Menge an Fibrin zusetzt oder diese in situ erzeugt und die zeitabhängige Trübungsänderung oder die zeitabhängige Bildung von Fibrin-Spaltprodukten mißt oder

b) ein chromogenes Plasminsubstrat und eine nicht zur Trübungsbildung ausreichende Menge an Fibrin, Fibrinogen-Spaltprodukten oder eines Fibrin in situ erzeugenden Enzyms zusetzt und die zeitabhängige Farbbildung mißt.

Unter Trübungsmessung wird hierbei im Rahmen der Erfindung verstanden die Messung der Trübungsabnahme oder -zunahme pro Zeiteinheit, die Messung der Zeit bis zum Erreichen des Trübungsmaximums oder die Messung der Zeit bis zum Erreichen einer bestimmten Trübungsabnahme oder -zunahme, bezogen auf das Trübungsmaximum. Auch eine Kombination dieser Meßmöglichkeiten kann angewendet werden. Besonders bevorzugt wird die Zeit bis zum Erreichen einer definierten Trübungsabnahme oder die Zeit bis zum Erreichen des Trübungsmaximums gemessen.

Die Farbmessung kann durch kinetische Verfolgung der Farbbildung erfolgen, wobei die Extinktionsänderung pro Zeiteinheit ermittelt wird. Ebenso kann die nach einer vorgegebenen Zeit erreichte Extinktion bestimmt werden oder es kann die Farbbildungsreaktion nach einer vorgegebenen Zeit abgestoppt und zu beliebiger Zeit später dann die gebildete Farbe gemessen werden.

Die Messung der gebildeten Fibrin-Spaltprodukte kann mit handelsüblichen Testreagenzien bzw. -Verfahren erfolgen. Beispiele sind Staphylokokken-clumping-Test (Hersteller Boehringer Mannheim GmbH) oder immunologische Verfahren z. B. über antikörperbeschichtete Latexpartikel (Hersteller Wellcome Corp.).

Das Abstoppen der Farbbildungsreaktion kann durch Zusatz geeigneter Inhibitoren, vorzugsweise Plasmininhibitoren oder Säuren wie z. B. Essigsäure oder Zitronensäure, erfolgen. Bevorzugt wird die Zeit bis zum Erreichen einer bestimmten Extinktion gemessen.

Das vorstehend definierte erfindungsgemäße Verfahren ergibt eine Bestimmung der hyperfibrinolytischen Aktivität des Plasmas. Dabei lösen bereits im Plasma vorhandene oder entstandene Plasminogenaktivatoren die Reaktion aus. Diese Ausführungsform des erfindungsgemäßen Verfahrens eignet sich besonders für die Verfolgung einer Fibrinolysetherapie. Man kann damit feststellen, wie sich der fibrinolytische Zustand des nativen Plasmas im Verlauf der Therapie ändert.

Gemäß einer weiteren Ausführungsform des erfindungsgemäßen Verfahrens wird außerdem eine bestimmte Menge an Plasminogenaktivator zugesetzt, wobei bevorzugt therapeutisch angewandte Konzentrationen eingesetzt werden. Bei dieser Ausführungsform des Verfahrens bestimmt man die mögliche Reaktivität des Plasmas in Gegenwart von definierten Mengen Plasminogenaktivator. Dies ist besonders wichtig für die Einleitung einer Fibrinolysetherapie. Man kann dabei feststellen, wie sich der fibrinolytische Zustand des Plasmas ändert, wenn die für eine Fibrinolysetherapie vorgesehenen Therapeutika wie t-PA (EPA), Urokinase oder Streptokinase, zugesetzt werden.

Gemäß einer bevorzugten Ausführungsform wird das Fibrin in situ durch Zusatz von Thrombin oder einem thrombinähnlichen Enzym wie Batroxobin oder Arvin erzeugt.

Als Fibrinogenspaltprodukte werden bevorzugt solche verwendet, die durch Behandlung von Fibrinogen mit Bromcyan erhalten werden (I. H. Verheijen, Thromb. Haemostas 48, 266-269 (1982). Bevorzugt werden Konzentrationen von 10 bis 150 µg/ml eingesetzt.

Fibrinmonomere werden hergestellt durch Behandlung von Fibrinogen mit Batroxobin, Inaktivierung des Batroxobins mit Diisopropylfluorophosphat, Entfernen und anschließendem Auflösen des Fibrinclots in 1- bis 3 molarer Harnstofflösung. Bevorzugt werden Konzentrationen von 1 bis 100 µg/ml Fibrinmonomer eingesetzt.

Als chromogenes Plasminsubstrat kann jedes Plasminsubstrat verwendet werden, aus welchem unter der Einwirkung von Plasmin eine zur Farbbildung geeignete Gruppe abgespalten wird. Als farbbildende Gruppen kommen hierbei sowohl solche in Frage, die direkt photometrisch gemessen werden können wie z. B. Nitroanilin, Dinitroanilin und deren Derivate als auch solche Verbindungen, die durch Reaktion mit einer weiteren Komponente eine Farbbildung bewirken. Beispiele hierfür sind:

Bei der Ausführungsform des erfindungsgemäßen Verfahrens unter Zusatz eines Plasminogenaktivators kann man als Plasminogenaktivator EPA (Extrinsic Plasminogen-Aktivator t-PA), Urokinase oder Streptokinase verwenden. Ein bevorzugtes chromogenes Plasminsubstrat ist Tos-Gly-Pro-Lys-p-Nitroanilin.

Das Verfahren der Erfindung kann bei einer beliebigen Temperatur zwischen etwa 20 und 40° C durchgeführt werden.

Ein weiterer Gegenstand der Erfindung ist ein Reagenz zur Bestimmung des fibrinolytischen Zustands von Plasma, welches für die Durchführung des Verfahrens der Erfindung geeignet ist. Ein derartiges Reagenz ist dadurch gekennzeichnet, daß es Plasminogenaktivator, Thrombin oder thrombinähnliches Enzym und Puffer enthält.

Anstelle von Thrombin oder thrombinähnlichem Enzym kann das Reagenz auch Fibrinspaltprodukte oder Fibrinmonomer enthalten.

Falls das erfindungsgemäße Reagenz für die Variante b) des erfindungsgemäßen Verfahrens, also die Farbmessung, bestimmt ist, enthält es zusätzlich ein chromogenes Plasminsubstrat.

Gemäß einer bevorzugten Ausführungsform enthält das erfindungsgemäße Reagenz außerdem wenigstens eine Substanz aus der Gruppe Polyethylenglykol, nichtionogenes, oberflächenaktives Mittel und Rinderserumalbumin.

In einer ersten Ausführungsform des erfindungsgemäßen Reagenz enthält dieses EPA, Batroxobin, Trispuffer pH 7 bis 8, Polyethylenglykol und nichtionogenes oberflächenaktives Mittel.

Für die Farbmessung enthält dieses Reagenz zweckmäßig anstelle des Batroxobins durch Bromcyanbehandlung von Fibrinogen hergestellte Fibrinogen-Spaltprodukte oder statt dessen Fibrinmonomer und außerdem Tos-Gly-Pro-Lys-p-Nitroanilin.

Für die einzelnen Bestandteile des erfindungsgemäßen Reagenz gelten die obigen Ausführungen im Zusammenhang mit der Erläuterung des Verfahrens entsprechend.

Die folgenden Beispiele erläutern die Erfindung weiter in Verbindung mit der beigefügten Zeichnung.

In dieser stellen dar:

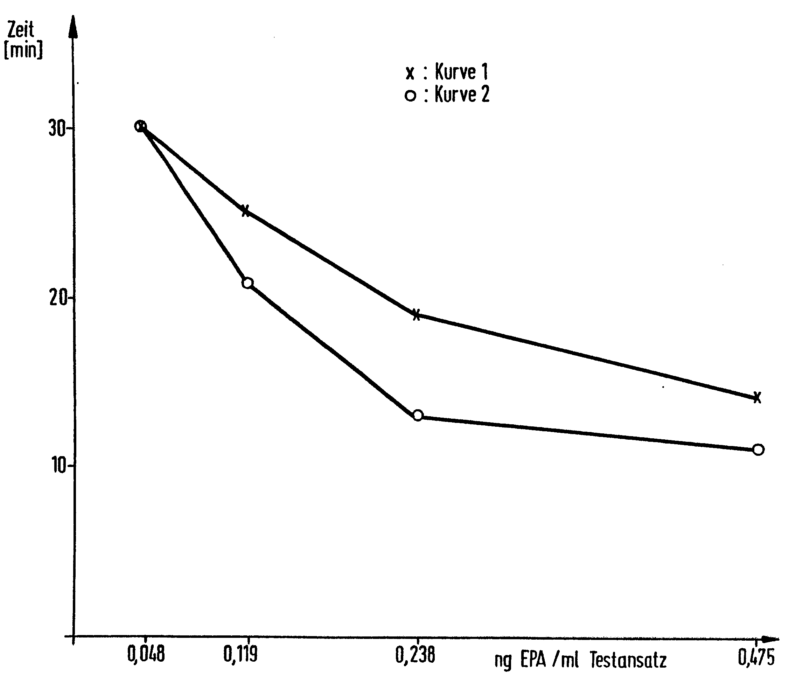

Fig. 1 eine Eichkurve für einen Trübungstest mit EPA als Plasminogenaktivator.

## Beispiel 1

Trübungstest mit EPA als Plasminogenaktivator

Reagenzzusammensetzung:
Tris/HCl, 0,1 mol/l, pH 7,5
Polyethylenglycol 6000, 2 Gew.-%
Oberflächenaktives Mittel (Tween 80), 0,1 Gew.-%
Rinderserumalbumin (RSA), 1 Gew.-%
Batroxobin 0,02 BU/ml
EPA (0,01 - 10 ng/ml Testansatz)

EP 0 189 910 B1

Testansatz:  50 µl Probe (Plasma) 1000 µl Reagenz

Reagenz und Probe werden bei 25°C gemischt und die Trübung sofort an einem Photometer bei $\lambda$ = 334 Nm verfolgt.

Die Extinktionsänderung wird mit einem Schreiber (Papiervorschub 0,1 cm/min) registriert.

Die Erstellung einer Eichkurve erfolgt entweder durch Erfassen der Zeit $t_1$ bis das Trübungsmaximum erreicht wird (Fig. 1, Kurve 1) oder der Zeit $t_2$ bis - ausgehend vom Trübungsmaximum - eine Extinktionsabnahme von 100 mE erfolgt ist (Fig. 1, Kurve 2).

**Beispiel 2**

Trübungstest mit Streptokinase bzw. Urokinase als Plasminogenaktivator.

Reagenzzusammensetzung, Testansatz und Testdurchführung entsprechen Beispiel 1, wobei anstelle von EPA Streptokinase bzw. Urokinase in unterschiedlichen Konzentrationen zugesetzt wird.

Es werden dabei folgende Meßwerte erhalten:

a) Streptokinase

| Konzentration iU/ml Testansatz | 0 | 0,05 | 0,10 | 0,15 | 0,20 | 0,25 | |
|---|---|---|---|---|---|---|---|
| $t_1$ [min] (vgl. Beispiel 1) | 55 | 40 | 36,5 | 17,0 | 15,0 | 10,5 | |
| $t_2$ [min] (vgl. Beispiel 1) | $\infty$ | 35 | 27 | 23 | 15 | 14 | |

b) Urokinase

| Konzentration ng/ml Testansatz | 0 | 10 | 50 | 100 | 200 | 300 | 400 |
|---|---|---|---|---|---|---|---|
| $t_1$ [min] | 65 | 50 | 23,5 | 16,5 | 12,0 | 9,5 | 8,5 |
| $t_2$ [min] | $\infty$ | 55 | 13 | 7,5 | 4,0 | 3,5 | 3,0 |

**Beispiel 3**

Farbtest mit EPA als Plasminogenaktivator

Reagenzzusammensetzung:
Tris/HCl 0,1 mol/l, pH 7,5
Polyethylenglycol 6000, 2 Gew.-%
Tween 80, 0,1 Gew.-%
RSA, 1 Gew.-%
EPA: 0,01 bis 10 ng/ml Testansatz
BrCN-Fragmente des Fibrins, 75 µg/ml
Plasminsubstrat TOS-Gly-Pro-Lys-pNA, 0,15 mmol/l

Testansatz:  50 µl Probe (Plasma) 1000 µl Reagenz

Reagenz und Probe werden bei 25°C gemischt und die Farbbildung an einem Photometer bei $\lambda$ = 405 nm verfolgt. Die Extinktionsänderung wird mit einem Schreiner (Papiervorschub 0,1 cm/min) registriert. Es wird die Zeit $t_3$ gemessen bis - ausgehend von der Basislinie (Reagenzleerwert) - ein Extinktionsanstieg von 100 mE erreicht ist.

Als Ergebnisse werden erhalten:

| Konzentration EPA ng/ml Testansatz | 0,048 | 0,119 | 0,238 | 0,475 |
|---|---|---|---|---|
| $t_3$ [min] | 118 | 58 | 36 | 24 |

**Patentansprüche**

1. Verfahren zur Bestimmung des fibrinolytischen Zustands von Plasma, dadurch gekennzeichnet,

daß man einer nativen Plasmaprobe

a) eine zur Trübungsbildung ausreichende Menge an Fibrin zusetzt oder diese in situ erzeugt und die zeitabhängige Trübungsänderung oder die zeitabhängige Bildung von Fibrin-Spaltprodukte mißt oder

b) ein chromogenes Plasminsubstrat und eine nicht zur Trübungsbildung ausreichende Menge an Fibrin, Fibrinogen-Spaltprodukten oder eines Fibrin in situ erzeugenden Enzyms zusetzt und die zeitabhängige Farbbildung mißt.

2. Verfahren nach Anspruch 1,
<u>dadurch gekennzeichnet,</u>
daß man außerdem eine bestimmte Menge Plasminogenaktivator zusetzt.

3. Verfahren nach Anspruch 1 oder 2,
<u>dadurch gekennzeichnet,</u>
daß man Fibrin in situ durch Zusatz von Thrombin oder einem thrombinähnlichen Enzym wie Batroxobin oder Arvin erzeugt.

4. Verfahren nach Anspruch 2 oder 3,
<u>dadurch gekennzeichnet,</u>
daß man als Plasminogenaktivator EPA, Urokinase oder Streptokinase zusetzt.

5. Verfahren nach einem der vorhergehenden Ansprüche,
<u>dadurch gekennzeichnet,</u>
daß man als Fibrinogen-Spaltprodukte solche verwendet, die durch Behandlung von Fibrinogen mit Bromcyan erhalten werden.

6. Verfahren nach einem der vorhergehenden Ansprüche,
<u>dadurch gekennzeichnet,</u>
daß man als chromogenes Plasminsubstrat Tos-Gly-Pro-Lys-p-Nitroanilin verwendet.

7. Reagenz zur Bestimmung des fibrinolytischen Zustands von Plasma nach dem Verfahren eines der Ansprüche 1 bis 6,
<u>dadurch gekennzeichnet,</u>
daß es Plasminogenaktivator, Thrombin oder thrombinähnliches Enzym und Puffer enthält.

8. Reagenz zur Bestimmung des fibrinolytischen Zustands von Plasma nach dem Verfahren eines der Ansprüche 1 bis 6,
<u>dadurch gekennzeichnet,</u>
daß es Plasminogenaktivator und Fibrinogenspaltprodukte oder Fibrinmonomer enthält.

9. Reagenz nach Anspruch 7 oder 8,
<u>dadurch gekennzeichnet,</u>
daß es zusätzlich ein chromogenes Plasminsubstrat enthält.

10. Reagenz nach den Ansprüchen 7 bis 9,
<u>dadurch gekennzeichnet,</u>
daß es Polyethylenglykol, ein nichtionogenes, oberflächenaktives Mittel und/oder Rinderserumalbumin enthält.

11. Reagenz nach einem der Ansprüche 7 bis 10,
<u>dadurch gekennzeichnet,</u>
daß es EPA, Batroxobin, Trispuffer pH 7 bis 8, Polyethylenglykol und nichtionogenes oberflächenaktives Mittel enthält.

12. Reagenz nach Anspruch 11,
<u>dadurch gekennzeichnet,</u>
daß es EPA, Trispuffer pH 7 bis 8, Polyethylenglycol, nichtionogenes oberflächenaktives Mittel und durch Bromcyanbehandlung von Fibrinogen hergestellte Fibrinogen-Spaltprodukte oder Fibrinmonomer und Tos-Gly-Pro-Lys-p-nitroanilin enthält.

13. Verwendung von Thrombin oder thrombinähnlichem Enzym zusammen mit Puffer als Reagenz zur Bestimmung des fibrinolytischen Zustands von Plasma nach dem Verfahren eines der Ansprüche 1 bis 6.

## Claims

1. Process for the determination of the fibrinolytic state of plasma, characterised in that to a native plasma sample one

a) adds an amount of fibrin sufficient for a turbidity formation or produces this <u>in situ</u> and measures the time-dependent turbidity change or the time-dependent formation of fission products or

b) adds a chromogenic plasmin substrate and an amount of fibrin, fibrinogen fission products or of an enzyme producing fibrin <u>in situ</u> insufficient for the turbidity formation and measures the time-dependent colour formation.

2. Process according to claim 1, characterised in that one also adds a definite amount of plasminogen activator.

3. Process according to claim 1 or 2, characterised in that one produces fibrin <u>in situ</u> by the addition of thrombin or of a thrombin-like enzyme, such as batroxobin or arvin.

EP 0 189 910 B1

4. Process according to claim 2 or 3, characterised in that one adds EPA, urokinase or streptokinase as plasminogen activator.

5. Process according to one of the preceding claims, characterised in that, as fibrinogen fission products, one uses those which are obtained by treatment of fibrinogen with cyanogen bromide.

6. Process according to one of the preceding claims, characterised in that, as chromogenic plasmin substrate, one uses Tos-Gly-Pro-Lys-p̲-nitroaniline.

7. Reagent for the determination of the fibrinolytic state of plasma according to a process of one of claims 1 to 6, characterised in that it contains plasminogen activator, thrombin or thrombin-like enzyme and buffer.

8. Reagent for the determination of the fibrinolytic state of plasma according to a process of one of claims 1 to 6, characterised in that it contains plasminogen activator and fibrinogen fission products or fibrin monomer.

9. Reagent according to claim 7 or 8, characterised in that it additionally contains a chromogenic plasmin substrate.

10. Reagent according to claims 7 to 9, characterised in that it contains polyethylene glycol, a non-ionic, surface-active agent and/or bovine serum albumin.

11. Reagent according to one of claims 7 to 10, characterised in that it contains EPA, batroxobin, tris buffer pH 7 to 8, polyethylene glycol and non-ionic, surface-active agent.

12. Reagent according to claim 11, characterised in that it contains EPA, tris buffer pH 7 to 8, polyethylene glycol, non-ionic, surface-active agent and fibrinogen fission products produced by cyanogen bromide or treatment of fibrinogen or fibrin monomer and Tos-Gly-Pro-Lys-p̲-nitroaniline.

13. Use of thrombin or thrombin-like enzyme, together with buffer, as reagent for the determination of the fibrinolytic state of plasma according to the process of one of claims 1 to 6.

## Revendications

1. Procédé pour la détermination de l'état fibrinolytique du plasma, caractérisé en ce qu'à un échantillon de plasma natif
a) on ajoute une quantité de fibrine suffisante pour la formation d'un trouble ou on produit celle-ci in situ et on mesure la modification du trouble dépendante du temps ou la formation, dépendante du temps, des produits de clivage de la fibrine ou
b) on ajoute un substrat chromogène de la plasmine et une quantité non suffisante pour former un trouble, de fibrine, de produits de clivage du fibrinogène ou d'une enzyme produisant de la fibrine in situ et on mesure la formation de couleur dépendante du temps.

2. Procédé selon la revendication 1, caractérisé en ce qu'on ajoute en outre une quantité déterminée d'activateur du plasminogène.

3. Procédé selon la revendication 1 ou 2, caractérisé en ce qu'on produit de la fibrine in situ par addition de thrombine ou d'une enzyme analogue à la thrombine, telle que la Batroxobine ou l'Arvine.

4. Procédé selon la revendication 2 ou 3, caractérisé en ce qu'on ajoute, en tant qu'activateur du plasminogène de l'EPA, de l'urokinase ou de la streptokinase.

5. Procédé selon l'une des revendications précédentes, caractérisé en ce qu'on utilise, en tant que produits de clivage de fibrinogène, des produits qui sont obtenus par traitement du fibrinogène par le bromure de cyanogène.

6. Procédé selon l'une des revendications précédentes, caractérisé en ce que l'on utilise, en tant que substrat chromogène de la plasmine, la Tos-Gly-Pro-Lys-p-nitroaniline.

7. Réactif pour la détermination de l'état fibrinolytique du plasma selon le procédé de l'une des revendications 1 à 6, caractérisé en ce qu'il contient de l'activateur du plasminogène, de la thrombine ou de l'enzyme analogue à la thrombine et du tampon.

8. Réactif pour la détermination de l'état fibrinolytique du plasma selon le procédé de l'une des revendications 1 à 6, caractérisé en ce qu'il contient de l'activateur de plasminogène et des produits de clivage du fibrinogène our de la fibrine monomère.

9. Réactif selon la revendication 7 ou 8, caractérisé en ce qu'il contient en outre un substrat chromogène de la plasmine.

10. Réactif selon les revendications 7 à 9, caractérisé en ce qu'il contient du polyéthylèneglycol, un agent tensioactif non ionogène et/ou de la sérumalbumine de bovidé.

11. Réactif selon l'une des revendication 7 à 10, caractérisé en ce qu'il contient de l'EPA, de la Batroxobine, du tampon Tris pH 7 à 8, du polyéthylèneglycol et de l'agent tensioactif non ionogène.

12. Réactif selon la revendication 11, caractérisé en ce qu'il contient de l'EPA, du tampon Tris pH 7 à 8, du polyéthylèneglycol, de l'agent tensioactif non ionogène et des produits de clivage du fibrinogène préparés par traitement du fibrinogène par le bromure de cyanogène ou de la fibrine monomère et de la Tos-Gly-Pro-Lys-p-nitroaniline.

13. Utilisation de la thrombine ou d'une enzyme analogue à la thrombine ensemble avec du tampon en tant que réactif pour la détermination de l'état fibrinolitique du plasma selon le procédé de l'une des revendication 1 à 6.

x : Kurve 1
o : Kurve 2

Zeit
[min]

30

20

10

0,048  0,119  0,238  0,475

ng EPA / ml  Testansatz

1